# EUROPEAN PATENT APPLICATION

(11) **EP 4 632 762 A1**
(43) Date of publication of application: **15.10.2025**
(21) Application number: 23901069.7
(22) Date of filing: 05.12.2023
(51) Int. Cl.: G16H 50/20, G16H 10/60, G16H 15/00, G16H 50/70, G16H 50/30, G06N 3/02, A61B 5/145, A61B 5/08

(54) **DEVICE AND METHOD FOR ASSISTING IN PREDICTING TIME OF EXTUBATION**

(30) Priority: 05.12.2022 KR 20220168057
(71) Applicant: Seoul National University Hospital, Seoul 03080 (KR)
(72) Inventor: SONG, Wongeun, Seongnam-si, Gyeonggi-do 13620 (KR); YOO, Sooyoung, Seongnam-si, Gyeonggi-do 13620 (KR); CHOI, Chang Won, Seongnam-si, Gyeonggi-do 13554 (KR); JUNG, Young Hwa, Seongnam-si, Gyeonggi-do 13620 (KR); BAEK, Hyunyoung, Seongnam-si, Gyeonggi-do 13620 (KR)
(74) Representative: Dentons Patent Solutions Rechtsanwaltsgesellschaft mbH
(86) International application number: PCT/KR2023/019922
(87) International publication number: WO 2024/123051

(57) **Abstract**

The present invention relates to an artificial intelligence-based method for assisting in predicting the probability of success of extubating a low-birth weight neonate or the time of extubating a low-birth weight neonate, the method comprising the steps of: acquiring information - including at least one of patient information, ventilation information, and vital sign information - about the low-birth weight neonate; calculating the probability of success of extubating the low-birth weight neonate by using a neural network model; and recommending performing extubation when the probability of success of extubation is at least a threshold value, and recommending keeping on an artificial respirator when the probability of success of extubation is smaller than the threshold value.

## Description

### Technical Field

The present application relates to a device and a method for assisting in predicting the time of extubation.

### Cross-Reference to Related Application

The present application claims priority to Korean Patent Application No. 10-2022-0168057, filed on December 5, 2022, the entire contents of which are incorporated herein by reference.

### Description of State-Funded Research and Development

This study was conducted with the support of the Ministry of Health and Welfare (Task Name: Data Labeling and Practical Clinical Study for Development of Emergency and Real-Time Diagnostic Artificial Intelligence Systems, Task No.: 1465036287, Detailed Task No.: HI18C0022020022)

### Background Art

Low-birth weight neonates require multiple sessions of mechanical ventilation (MV) to support their immature lungs due to immature lung development. However, more than necessary intubation can cause problems, such as oxygen toxicity, bronchopulmonary dysmorphia, and neurodevelopmental disorders, and thus extubation must be performed as early as possible. For extubation, various clinical information must be synthesized and complex causal relationships must be considered. There is still no internationally agreed extubation protocol and the probability and specificity of successful extubation vary depending on the availability of medical information and medical staff.

Previously, spontaneous breathing trials (SBT) were used to determine whether extremely low-birth weight neonates were ready for extubation. However, the SBT showed low accuracy with one-third of failed extubation. In addition, low-birth weight neonates generally experience side effects during SBT. Few studies have investigated the dynamics of physiological signals as a predictive indicator of readiness for ventilation or extubation in low-birth weight neonates. Using physiological signals together with SBT, changes in heart rate or respiratory rate only slightly improved the capability to predict success or failure of extubation in small studies.

### Detailed Description of the Invention

### Technical Solution

In order to solve the above problems, the present application provides an accurate and field-applicable prediction model that identifies physiological signals separately from SBT and calculates the possibility of readiness for extubation based on the physiological signals in order to distinguish the predicted success or failure of extubation.

Specifically, the present application is largely composed of a prediction model (neural network model) and a result analysis to determine whether to extubate a low-birth weight neonate. For the prediction model, demographic information, such as gestational age and birth weight of an intubated neonate, vital sign information, such as oxygen saturation measured and recorded periodically, hourly variance and fluctuation of heart rate and respiratory rate, records of ventilation information, such as fraction of inspired oxygen (FiO2), mean arterial pressure (MAP), and positive end expiratory pressure (PEEP), and an SpO2/FiO2 (SF) ratio and a ratio of oxygen saturation (ROX) index, which are biomarkers reflecting the effect of the neonate's artificial respirator, are used. A classifier of the prediction model may apply a complement naive Bayesian model and a logistic regression model that can achieve uniform performance at a high degree of imbalance of data. The analysis of the results numerically and visibly provides the rationale and probability on which the prediction model derived the success rate using the theoretically verified SHAP (SHapley Additive exPlanations).

### Summary of the Invention

According to one embodiment of the present application, an artificial intelligence-based method for assisting in predicting the probability of success of extubating a low-birth weight neonate or the time of extubating a low-birth weight neonate, performed by a processor, the method comprising the steps of: acquiring information - including at least one of patient information, ventilation information, and vital sign information - about the low-birth weight neonate; calculating the probability of success of extubating the low-birth weight neonate by using a neural network model.

In one embodiment, the method further comprises: recommending performing extubation when the probability of successful extubation is at least a threshold value; recommending keeping on an artificial respirator when the probability of successful extubation is smaller than the threshold value, wherein the threshold value may refer to a minimum point at which extubation of the low-birth weight neonate is recommended.

In one embodiment, the method may further comprise: interpreting a result of the calculated probability of successful extubation to provide a report.

In one embodiment, the interpreting of the result to provide the report may include: numerically providing information on prediction parameters from which the neural network model calculates the probability of successful extubation; and graphically visualizing and providing a contribution of the prediction parameters.

In one embodiment, the graphically visualizing and providing of the contribution of the prediction parameters may include providing, in a hitmap format, an amount of change in the prediction parameter values from a specific point in the past to a present point in time.

In one embodiment, the interpreting of the result to provide the report may include graphically visualizing and providing the probability of successful extubation from a specific point in the past to a present point in time.

In one embodiment, the prediction parameters may be at least one or more of a heart rate, a respiratory rate, a body temperature, an oxygen saturation (SpO2), a blood pressure, gestational age (GA), a birth weight, a postmenstrual age (PMA) at extubation, a male, pre-extubation blood gas (pH, pCO2), artificial respirator setting information including inspiratory oxygen fraction (FiO2), positive end expiratory pressure (PEEP), mean arterial pressure (MAP), frequency, SpO2/FiO2 (SF) ratio, ratio of SpO2/FiO2 to respiratory rate (ROX), and respiratory severity score (RSS) obtained by multiplying MAP by FiO2.

In one embodiment, among the prediction parameters, a prediction parameter that contributes the most to the neural network model's calculation of the probability of successful extubation may be an SF ratio.

In one embodiment, the interpreting of the result of the calculated probability of successful extubation may include calculating, interpreting, and analyzing the importance of the feature extracted by the neural network by applying the SHAP to evaluate the interpretability of potential prediction parameters.

In one embodiment, the neural network model may be one of the logistic regression model and the complement naive Bayesian model.

In one embodiment, the method for assisting in predicting the probability of successful extubation may be implemented by a computer program stored in a computer-readable recording medium.

According to another embodiment of the present application, there is provided an artificial intelligence-based device for assisting in predicting the probability of success of extubating a low-birth weight neonate or predicting the time of extubating a low-birth weight neonate, where the device may include: an acquisition unit configured to acquire information on the low-birth weight neonate, including one or more of patient information, ventilation information, and vital sign information; and a neural network model configured to calculate the probability of success of extubating the low-birth weight neonate by using the information on the low-birth weight neonate as an input.

In one embodiment, the device may further include an interpretation unit that interprets the result of the calculated probability of successful extubation to provide a report.

In one embodiment, the interpretation unit may numerically provide information on prediction parameters from which the neural network model calculates the probability of successful extubation or may graphically visualize and provide the contribution of the prediction parameters.

### Effects of Invention

According to a device and a method for assisting in predicting the time for extubating a low-birth weight neonate, the success rate of extubation of MV, which is difficult to predict, based on hospitalization information and vital signs of the low-birth weight neonate, is predicted and the contributing factors thereof is analyzed, thereby assisting medical staff in making extubation decisions and suggesting the time of extubating the low-birth weight neonate. Accordingly, the present application provides medical staff with the success rate of extubation and predictive indicators to assist in evidence-based extubation decision. This aims to reduce the mortality and prevalence of low-birth weight neonates due to inadequate extubation.

The effects of the present application are not limited to the effects mentioned above, and other effects not mentioned may be clearly understood by a person skilled in the art from the description of the claims.

### Brief Description of the Drawings

In order to describe the technical solutions of embodiments of the present application or the prior art more clearly, the drawings required for the description of the embodiments are briefly introduced below. It shall be understood that the following drawings are only for the purpose of describing embodiments of the present application and are not intended to limit the same. In addition, some elements may be shown with various modifications, such as exaggerations, omissions, etc., in the following drawings for clarity of description.
FIG. 1 is a flowchart of a method for assisting in predicting the time of extubation, according to an embodiment of the present application.
FIG. 2 is a detailed flowchart of interpreting a prediction result to provide a report, according to an embodiment of the present application.
FIG. 3 is a table showing details of obtained information on a low-birth weight neonate, according to an embodiment of the present application.
FIG. 4 is a table for multivariate analysis of potential variables for predicting successful extubation, according to an embodiment of the present application.
FIGS. 5A and 5B are PDP plots for analyzing the correlation between an SF ratio and a calculated probability of successful extubation, according to an embodiment of the present application, where FIG. 5A shows an average of the SF ratio and FIG. 5B shows an average of sequential differences of the SF ratio.
FIGS. 6A to 6H are images illustrating steps of numerically providing information on prediction parameters and graphically visualizing and providing a contribution of prediction parameters in interpreting a prediction result, according to an embodiment of the present application.
FIG. 7 is a block diagram of configurations in which the present application performs a main function when implemented in software, according to an embodiment of the present application.

### Detailed Description of the Embodiments

The terminology used herein is for the purpose of referring only to specific embodiments and is not intended to limit the present application. As used herein, the singular forms "a", "an", and "the" include plural forms unless the context clearly dictates otherwise. The meaning of "comprising," as used herein, embodies particular features, regions, integers, steps, operations, items, and/or components, and does not preclude the presence or addition of other features, regions, integrals, steps, operation, items, and or components.

Although not defined otherwise, all terms including technical and scientific terms used herein have the same meaning as commonly understood by a person skilled in the art to which the present application belongs. The commonly used predefined terms are further interpreted as having a meaning consistent with the relevant technical literature and the currently disclosed content and are not interpreted in an ideal or very official meaning unless defined.

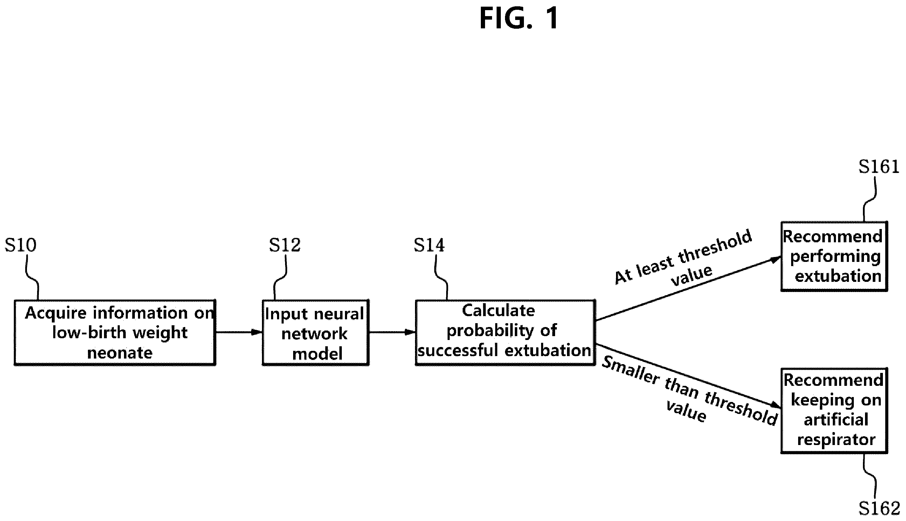

FIG. 1 is a flowchart of a method for assisting in predicting the time of extubation, according to an embodiment of the present application.

Referring to FIG. 1, the method for assisting in predicting the time of extubation includes: acquiring information on a low-birth weight neonate (S10); inputting the information on the low-birth weight neonate into a neural network model (S12); calculating the probability of success of extubating the low-birth weight neonate (S14); and recommending performing extubation when the calculated probability of successful extubation is at least a threshold value (S161), or recommending keeping on an artificial respirator when the calculated probability of successful extubation is smaller than the threshold value (S162). The information on the low-birth weight neonate may include one or more of demographic information, ventilation information, and vital sign information. The threshold value refers to a minimum point at which the low-birth weight neonate is recommended to perform extubation. In one example, the threshold value may be set to 0.98.

As the neural network model, various artificial intelligence models including logistic regression, complementary naive Bayesian model, random forest, gradient boosting, decision tree, stochastic gradient descent (SGD) classifier, extreme gradient boosting (XGB), and the like can be used. In particular, in the neural network model of the present application, a logistic regression model and a complement naive Bayesian model that can derive uniform performance at a high degree of imbalance of data can be used.

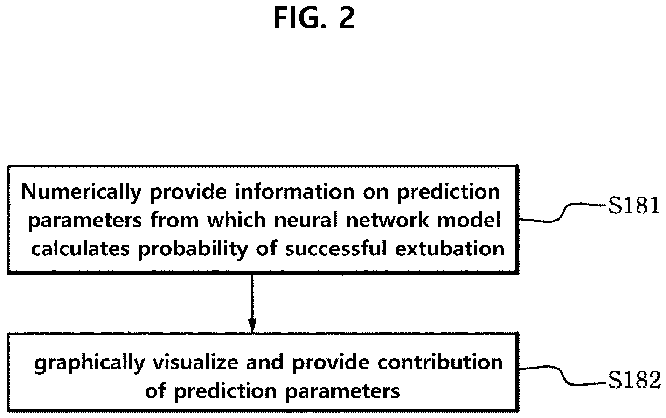

FIG. 2 is a detailed flowchart of interpreting a prediction result to provide a report, according to an embodiment of the present application.

Referring to FIG. 2, the method for assisting in predicting the time of extubation may further include interpreting the result of calculating the probability of successful extubation to provide a report (S18). The interpreting of the result of calculating the probability of successful extubation to provide a report (S18) may include numerically providing information on prediction parameters from which the neural network model calculates the probability of successful extubation (S181); and graphically visualizing and providing a contribution of the prediction parameters (S182). The prediction parameters may be at least one or more of a heart rate, a respiratory rate, a body temperature, an oxygen saturation (SpO2), a blood pressure, a gestational age (GA), a birth weight, a postmenstrual age (PMA) at extubation, male, blood gas before extubation (pH, pCO2), artificial respirator setting information including oxygen fraction supplied from the artificial respirator (FiO2), a positive end expiratory pressure (PEEP), a mean arterial pressure (MAP), a set respiratory frequency of the artificial respirator, SpO2/FiO2 (SF) ratio, a ratio of SpO2 /FiO2 to respiratory rate (ROX), and respiratory severity score (RSS) obtained by multiplying MAP by FiO2. In particular, among the prediction parameters, a prediction parameter that contributes the most to the neural network model's calculation of the probability of successful extubation may be the SF ratio. The interpreting of the result of the calculated probability of successful extubation may include calculating, interpreting, and analyzing the importance of features extracted by the neural network by applying SHapley Additive exPlanation (SHAP) to evaluate the interpretability of potential prediction parameters.

The graphically visualizing and providing of the contribution of the prediction parameters may include providing, in a hitmap format, an amount of change in the prediction parameter values from a specific point in the past to a present point in time. The interpreting of the result to provide a report may include graphically visualizing and providing the probability of successful extubation from a specific point in the past to the present point in time. In one example, the specific point in the past may be 24 hours before the present point in time.

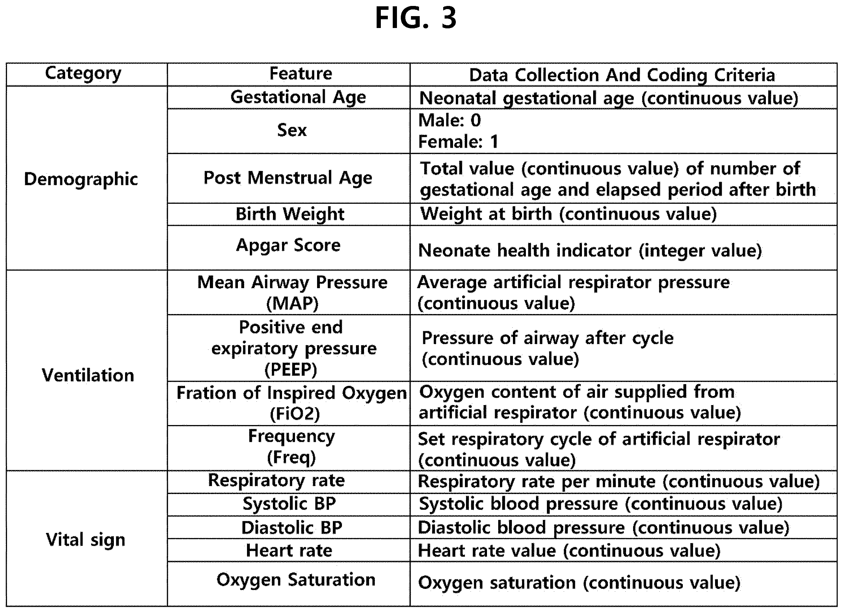

FIG. 3 is a table showing details of obtained information on a low-birth weight neonate, according to an embodiment of the present application.

Referring to FIG. 3, in the acquiring of information on the low-birth weight neonate (S10), the information on the low-birth weight neonate may include one or more of demographic information, ventilation information, and vital sign information. In one embodiment, the demographic information may include gestational age, sex, birth weight, and Apgar score. Based on data collection and coding criteria, the gender may be set to 0 in the case of male and may be set to 1 in the case of female, and the Apgar score, as a neonatal health index, may be set to an integer value.

The ventilation information may include a MAP which is an average pressure of the artificial respirator, a PEEP which is a pressure of the airway after exhalation, a fraction of inspired oxygen (FiO2) which is an oxygen content of air supplied from the artificial respirator, and a frequency (Freq) which is a set breathing cycle of the artificial respirator.

The vital sign information may include a respiratory rate (RR), systolic BP, diastolic BP, heart rate, and oxygen saturation.

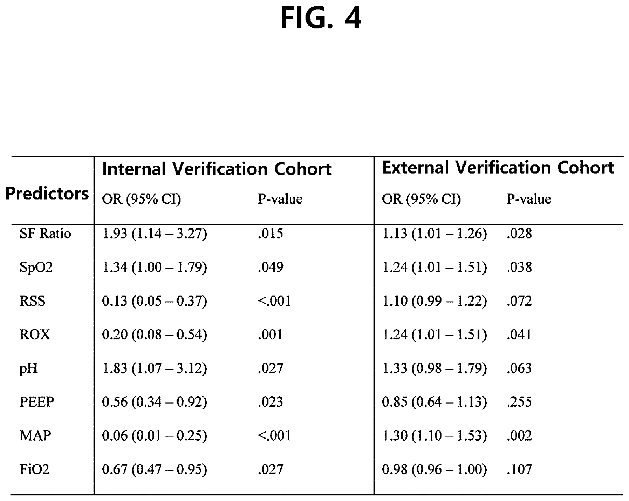

FIG. 4 is a table for multivariate analysis of potential variables for predicting successful extubation, according to an embodiment of the present application.

Referring to FIG. 4, the SF ratio analyzed by SHAP and PDP was a significant predictive indicator of successful extubation in all cohorts. Of potential variables, only the SF ratio and SpO2 were definite predictive indicators of successful extubation. Specifically, FiO2, PEEP, and RSS, which were predominantly included in other studies, predicted success or failure of extubation in the internal validation cohort but not in the external validation cohort. MAP, SpO2 and SF ratio significantly differentiated between success and failure of extubation in the external validation cohort, but only SF ratio and SpO2 were significant predictive indicators of successful extubation in multivariate analysis.

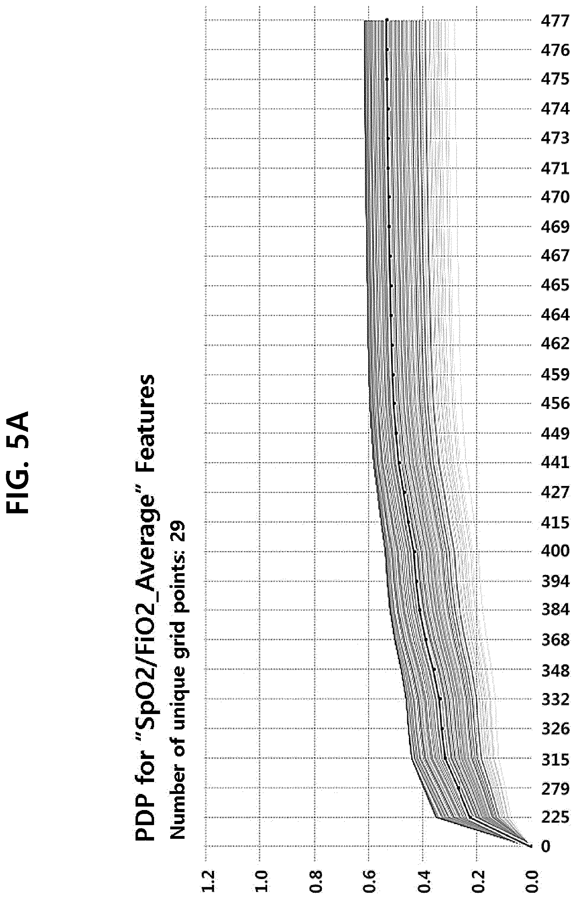

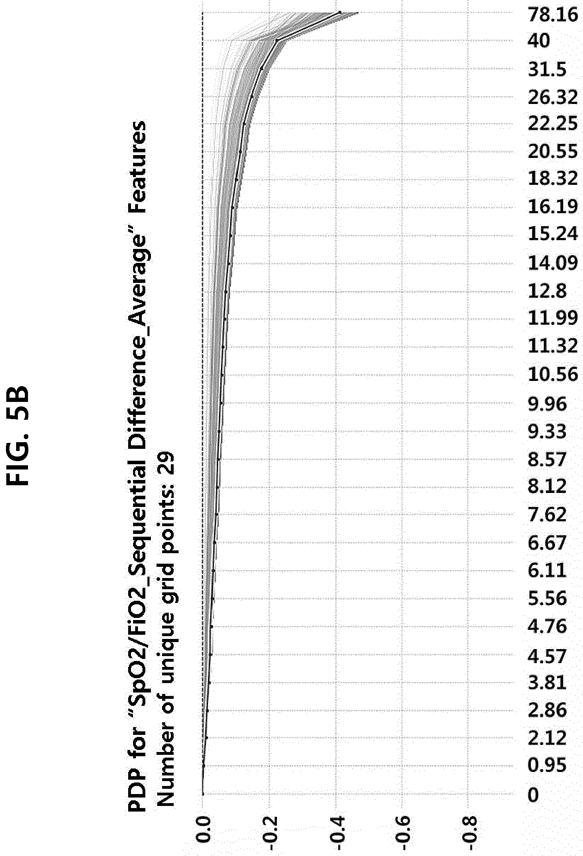

FIGS. 5A and 5B are PDP plots for analyzing the correlation between an SF ratio and a calculated probability of successful extubation, according to an embodiment of the present application, where FIG. 5A shows an average of the SF ratio and FIG. 5B shows an average of sequential differences of the SF ratio.

Referring to FIGS. 5A and 5B, as the sequential difference mean of the SF ratio increased, the probability of successful extubation showed a negative correlation. The mean of SF ratio and the sequential difference mean of SF ratio were identified as significant predictive indicators of successful extubation. The SF ratio had uniformly high classification for all cohorts when analyzed using SHAP and PDP. The SHAP value of the sequential difference mean of the SF ratio was 0.32, indicating the highest ratio. The present application predicts the probability of success or failure of extubation with only the SF ratio, compared to the prior art which used the birth weight and gestational age as the main determinants of success or failure of extubation.

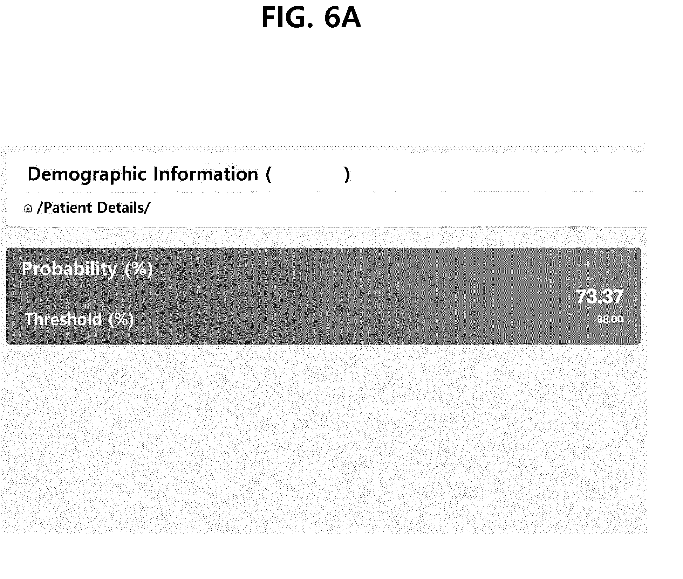

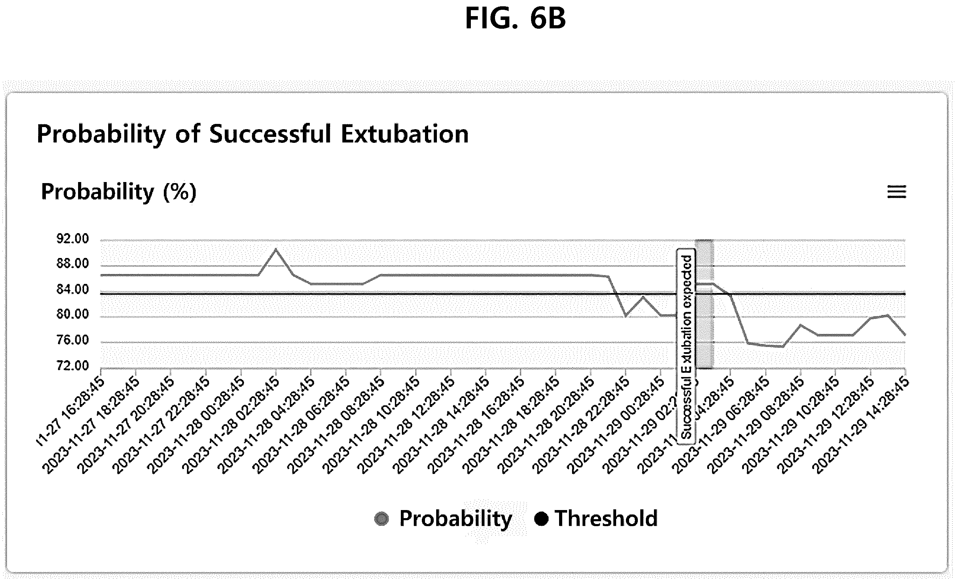

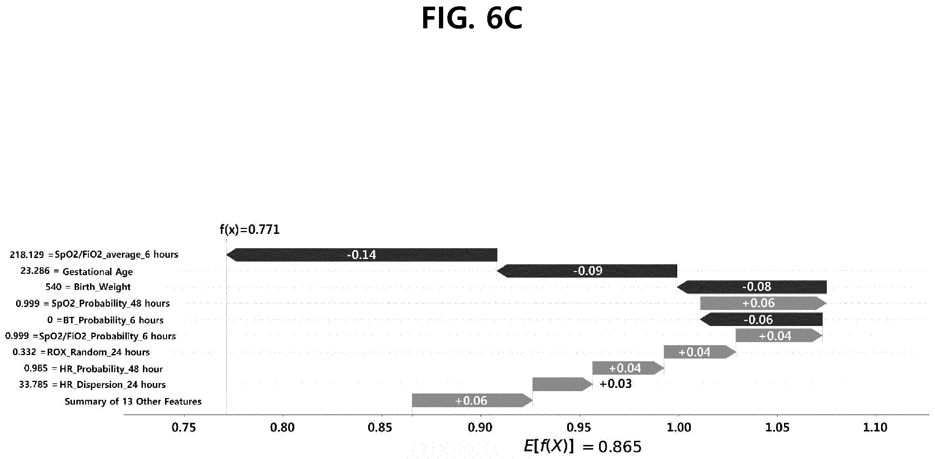

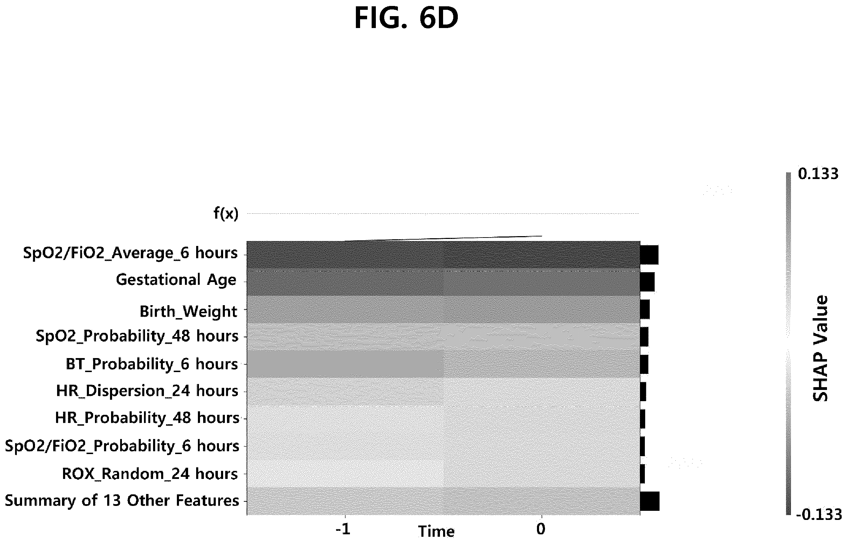

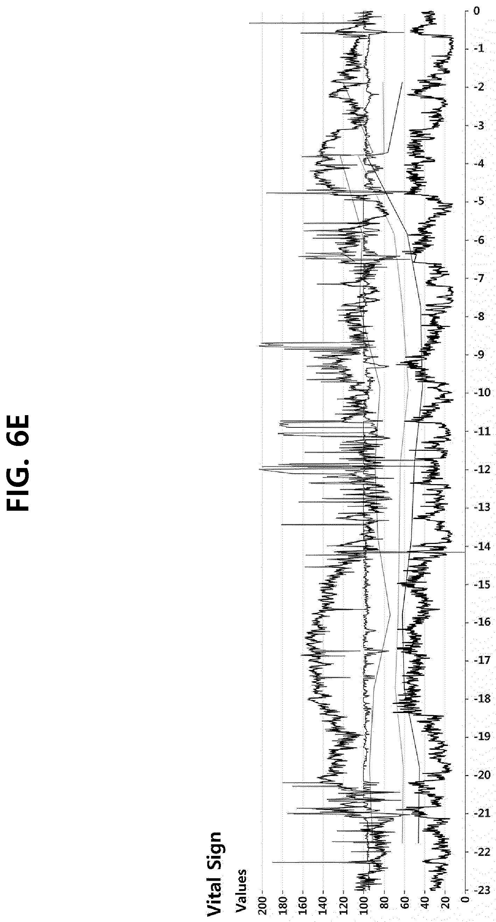

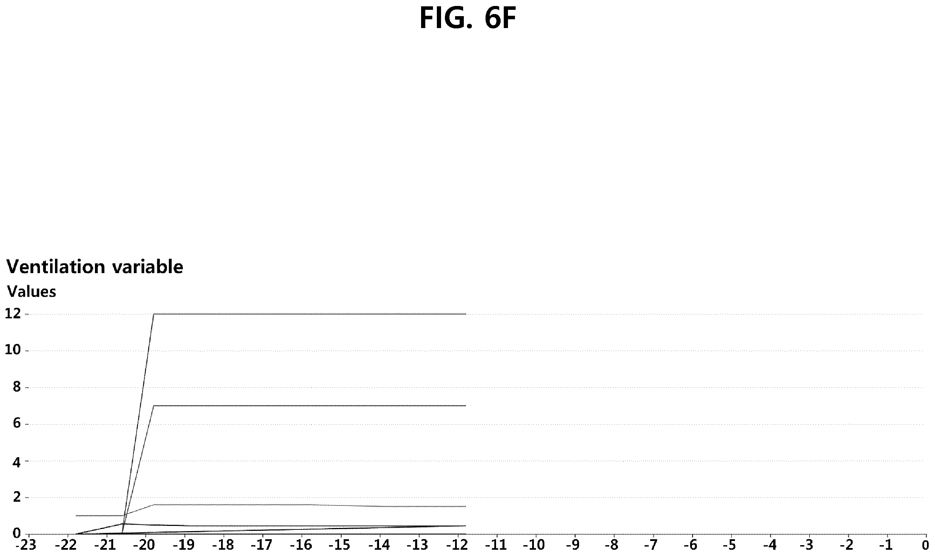

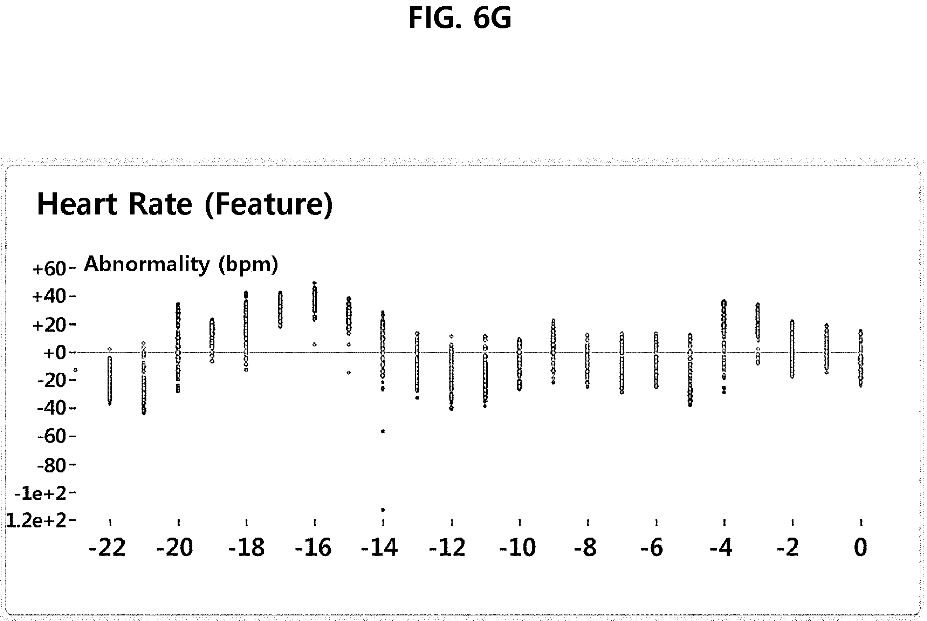

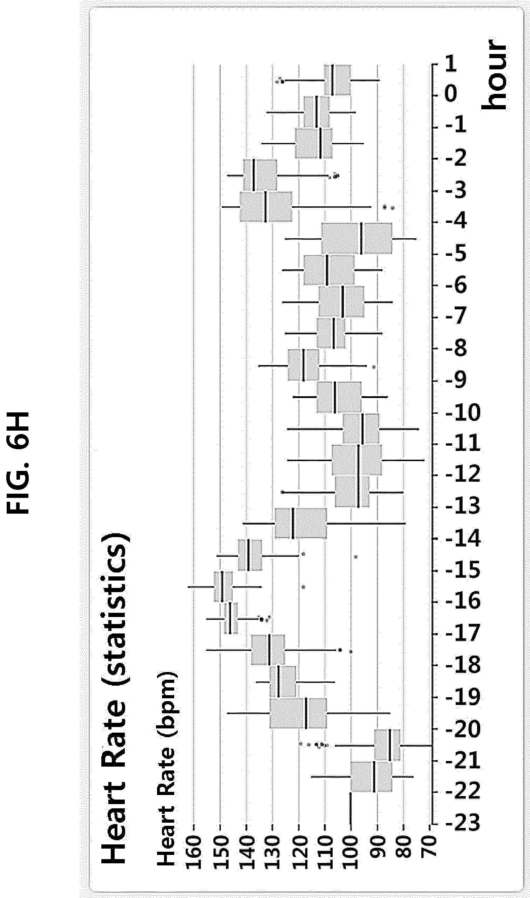

FIGS. 6A to 6H are images illustrating steps of numerically providing information on prediction parameters and graphically visualizing and providing a contribution of prediction parameters in interpreting a prediction result, according to an embodiment of the present application.

A method of numerically providing the information on prediction parameters and graphically visualizing and providing the contribution of the prediction parameters may be displayed on an output device such as a display or a speaker. As another example, an analysis result may be provided to a device in which functions for input and output are integrated into one, such as a touchscreen.

Referring to FIGS. 6A to 6H, the method of numerically providing the information on prediction parameters and graphically visualizing and providing the contribution of the prediction parameters may include: displaying, on the output device, a name and an identifier of a patient whose details are to be disclosed (1); and displaying the patient's success rate of extubation (2), and the threshold value that is a minimum point at which the patient is recommended to perform extubation (3). In addition, the method may include displaying in the form of a timeline that visualizes the success rate of extubation from a specific point in the past (e.g., 24 hours ago from the present point in time) to the present point in time (4); and displaying in the form of a factor contribution graph showing the SHAP analysis result at the current prediction time (5). In addition, The method includes displaying in the form of a graph showing the change in SHAP values by factor from a specific point in the past to the present point in time in the form of a heatmap (6), a graph showing bio-signal values from a specific point in the past to the present point in time (7), a graph showing the amount of change in ventilator setting information from a specific point in the past to the present point in time (8), a graph showing outlier values of bio-signals (9), and a box plot showing statistical summary information of bio-signals for each time unit.

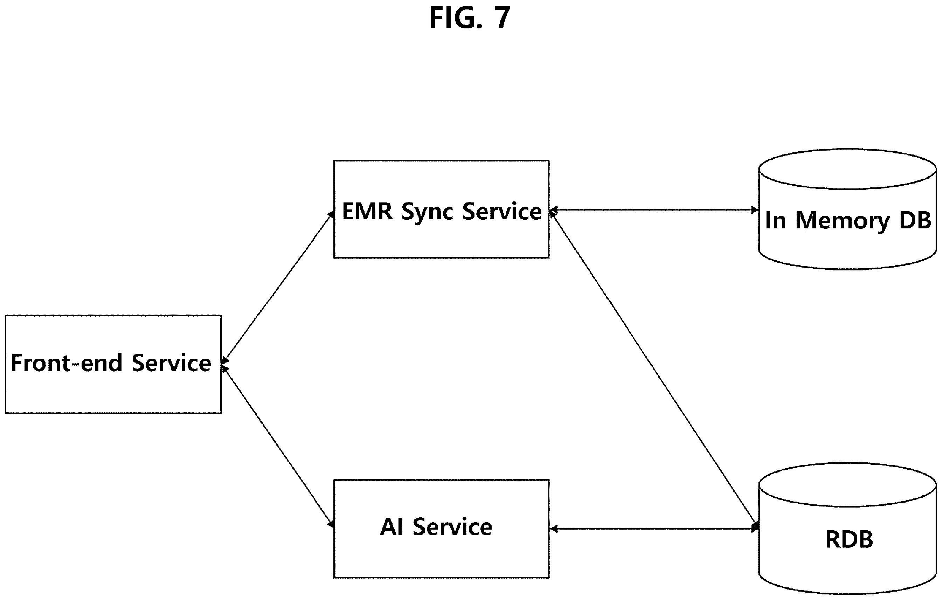

FIG. 7 is a block diagram of configurations in which the present application performs a main function when implemented in software, according to an embodiment of the present application.

Referring to FIG. 7, the present application may include a front-end service, an EMR interworking service, an AI service, a relational DB (RDB), an in-memory DB, and the like. The front-end service includes an authentication module, a WebAPI module, a settings management module, and a DB connection module. The EMR interworking service includes a raw data analysis module, a data monitoring module, and a DB connection module. The AI service includes an AI module, a DB connection module, and a factor analysis module. The detailed structure thereof is as follows.

The authentication module is used to confirm software access rights and execution, such as login, membership registration, administrator account, current session rights, and time limits.

The WebAPI module is used to provide front-end functions such as webpage access, HTTP protocol communications for users to use software. The WebAPI module shows the information on a hospitalized patient through a patient list, and allows a user to query the hospitalized patient information and the biosignal information, query the patient's details and past records, input and update the patient's ventilation information, and query the success rate of extubation based on the information input by the user and the patient data.

The settings management module is used to reflect and manage settings for service execution, such as a host, a port, a log setting, and a database URI. The settings management module sets the URI information on the DB to be used, sets the log level and the output method, and sets the allowed IP band and the port to set the web connection.

The DB connection module is used for the service to perform functions of data lookup, modification, deletion, and update.

The raw data analysis module is a module that converts and analyzes original data in which patient information and biosignals are recorded into a processable data format. The raw data analysis module parses the source data into a processable object, verifies whether the input data is valid, and determines what type of format (patient information, biosignals, and lab data) the source data is for.

The data monitoring module is a module for identifying whether original data is added and updated. The data monitoring module may query whether to add or update data in a set period, and if there is updated or added data, the data monitoring module may transmit the updated or added data to the in-memory DB to query a connected service. In addition, data is converted into a linkable json format.

The AI module is used to load the prediction model, which is a neural network model, and input patient information into the prediction model to calculate a result of predicting successful extubation.

The factor analysis module is used to analyze the interpretation information to provide the result calculated by the AI module to the medical staff by using SHAP or the like. Specifically, the factor analysis module loads the SHAP explainer to interpret the result of a registration model so that the result of the neural network model can be used at the front end, and uses the explainer to produce information about the contribution of factors to the prediction result.

The software may include a computer program, code, instructions, or a combination of one or more thereof, and may configure or independently or collectively instruct a processing device to operate as desired. The software and/or data may be embodied in any type of machine, component, physical device, virtual equipment, computer storage medium or device for interpretation by the processing device or for providing instructions or data to the processing device. The software may be distributed across a networked computer system and stored or executed in a distributed manner. The software and data may be stored in one or more computer-readable recording media.

According to another aspect of the present application, a method for assisting in predicting the time of extubation may be performed by a computing device including a processor. For example, each of the acquiring information - including at least one of patient information, ventilation information, and vital sign information - on a low-birth weight neonate (S10), inputting the information on the low-birth weight neonate into a neural network model (S12); calculating the probability of success of extubating the low-birth weight neonate (S14); and interpreting a result of calculating the probability of successful extubation to provide a report (S18) may be performed by an acquisition unit, a neural network model and an interpretation unit, which are computing devices including a processor. The computing device may be a computing device, such as a desktop computer, a laptop computer, a notebook computer, a smartphone, or the like, or any device that can be integrated. A computer is a device having one or more general and special purpose processors, memory, storage, and networking components (either wireless or wired). The computer may, for example, run an operating system, such as an operating system compatible with Microsoft's Windows, Apple OS X or iOS, Linux distribution, or Google's Android OS.

The operations by the method of assisting in predicting the time of extubation according to the embodiments described above may be at least partially implemented in a computer program and recorded in a computer-readable recording medium. For example, the operations may be implemented with a program product composed of a computer-readable medium comprising program code, which can be executed by a processor for performing any or all of the steps, operations, or processes described.

The computer-readable recording medium includes all kinds of recording identification devices in which data that can be read by a computer is stored. Examples of the computer-readable recording medium include ROM, RAM, CD-ROM, a magnetic tape, a floppy disk, an optical data storage identification device, and the like. In addition, the computer-readable recording medium may be distributed in a network-connected computer system, and computer-readable code may be stored and executed in a distributed manner. In addition, functional programs, code, and code segments for implementing the present embodiment may be easily understood by a person skilled in the art to which the present embodiment belongs.

According to the device and the method for assisting in predicting the time of extubation, the success rate of extubation of MV, which is difficult to predict, based on hospitalization information and vital signs of the low-birth weight neonate, is predicted and the contributing factors thereof is analyzed, thereby assisting medical staff in making extubation decisions and suggesting the time of extubating the low-birth weight neonate. Accordingly, the present application aims to provide medical staff with the success rate of extubation and the predictive indicators to assist in evidence-based extubation decision. The present invention also aims to lower the mortality and prevalence of low-birth weight neonate through adequate extubation.

Although the present application has been described above with reference to the embodiments shown in the drawings, it is merely illustrative. Those skilled in the art will understand that various modifications and variations of the embodiments are possible therefrom. However, such modifications should be considered to be within the technical protection scope of the present application. Therefore, the true technical protection scope of the present application should be determined by the technical idea of the appended claims.

### Industrial Applicability

A device and a method for assisting in predicting the time of extubation in embodiments of the present application predicts the success rate of extubation of MV, that is difficult to predict, based on hospitalization information and biosignals of a low-birth weight neonate, and provides analysis of the factor contribution thereto to assist a medical staff in determining extubation and proposes the time of extubating the low-birth weight neonate. Accordingly, the present application provides medical staff with the success rate of extubation and predictive indicators to assist in evidence-based extubation decision. This aims to reduce the mortality and prevalence of low-birth weight neonate through inadequate extubation.

## Claims

1. An artificial intelligence-based method for assisting in predicting the probability of success of extubating a low-birth weight neonate or the time of extubating a low-birth weight neonate, performed by a processor, the method comprising:
acquiring information on the low-birth weight neonate, including one or more of patient information, ventilation information, and vital sign information; an calculating, by using a neural network model, the probability of success of extubating the low-birth weight neonate.

2. The method of claim 1, further comprising recommending performing extubation when the probability of successful extubation is at least a threshold value, and
recommending keeping on an artificial respirator when the probability of successful extubation is less than a threshold value, wherein the threshold value refers to a minimum point at which the low-birth weight neonate is recommended to perform extubation.

3. The method of claim 1, further comprising interpreting the result of the calculated probability of successful extubation to provide a report.

4. The method of claim 3, wherein the interpreting of the result to provide a report comprises numerically providing information on prediction parameters by which the neural network model calculates the probability of successful extubation, and graphically visualizing and providing the contribution of the prediction parameters.

5. The method of claim 4, wherein the graphically visualizing and providing of the contribution of the prediction parameters comprises providing, in a hitmap format, an amount of change in the prediction parameter values from a specific point in the past to a present point in time.

6. The method of claim 3, wherein the interpreting of the result to provide a report comprises graphically visualizing and providing the probability of successful extubation from a specific point in the past to a present point in time.

7. The method of claim 4, wherein the prediction parameters are at least one or more of a heart rate, a respiratory rate, a body temperature, an oxygen saturation (SpO2), a blood pressure, gestational age (GA), a birth weight, a postmenstrual age (PMA) at extubation, male, blood gas before extubation (pH, pCO2), artificial respirator setting information including inspiratory oxygen fraction (FiO2), a positive end expiratory pressure (PEEP), a mean arterial pressure (MAP), a frequency, a SpO2/FiO2 (SF) ratio, a ratio of SpO2 /FiO2 to respiratory rate (ROX), and a respiratory severity score (RSS) obtained by multiplying MAP by FiO2.

8. The method of claim 7, wherein, among the prediction parameters, a prediction parameter that contributes the most to the neural network model's calculation of the probability of successful extubation is the SF ratio.

9. The method of claim 3, wherein the interpreting of the result of the calculated probability of successful extubation comprises computing, interpreting, and analyzing the importance of features extracted by the neural network by applying SHapley Additive exPlanation (SHAP) to evaluate the interpretability of potential prediction parameters.

10. The method of claim 1, wherein the neural network model comprises one of a logistic regression model and a complement naive Bayesian model.

11. A computer program stored in a computer-readable recording medium for implementing the method for assisting in predicting the probability of success of extubating a low-birth weight neonate according to any one of claims 1 to 10.

12. An artificial intelligence-based device for assisting in predicting the probability of success of extubating a low-birth weight neonate or predicting the time of extubating a low-birth weight neonate, the device comprising:
an acquisition unit for acquiring information on a low-birth weight neonate including one or more of patient information, ventilation information, and vital sign information; and
a neural network model for calculating the probability of success of extubating the low-birth weight neonate by using the information on the low-birth weight neonate as an input.

13. The device of claim 12, further comprising an interpretation unit for analyzing a result of calculating the probability of success of extubating the low-birth weight neonate to provide a report.

14. The device of claim 13, wherein the interpretation unit numerically provides information on prediction parameters from which the neural network model calculates the probability of successful extubation, or graphically visualizes and provides the contribution of the prediction parameters.

15. The device of claim 14, wherein the prediction parameters are at least one or more of a heart rate, a respiratory rate, a body temperature, an oxygen saturation (SpO2), a blood pressure, gestational age (GA), a birth weight, a postmenstrual age (PMA) at extubation, male, blood gas before extubation (pH, pCO2), artificial respirator setting information including inspiratory oxygen fraction (FiO2), a positive end expiratory pressure (PEEP), a mean arterial pressure (MAP), a frequency, a SpO2/FiO2 (SF) ratio, a ratio of SpO2 /FiO2 to respiratory rate (ROX), and a respiratory severity score (RSS) obtained by multiplying MAP by FiO2.

16. The device of claim 15, wherein, among the prediction parameters, a prediction parameter that contributes the most to the neural network model's calculation of the probability of successful extubation is the SF ratio.
